# FASCICULE DE BREVET EUROPEEN

(11) **EP 0 657 179 B1**
(45) Date de publication et mention de la délivrance du brevet: **27.10.1999**
(21) Numéro de dépôt: 94402766.3
(22) Date de dépôt: 02.12.1994
(51) Int. Cl.: A61M 1/34, G01N 33/49

(54) **Procédé et dispositif pour mesurer la concentration d'au moins une substance contenue dans un milieu complexe**
Gerät und Verfahren zur Messung der Konzentration von mindestens einem Bestandteil in einem komplexen flüssigen Medium
Device and procedure for measuring the concentration of at least one substance in a complex liquid medium

(30) Priorité: 06.12.1993 FR 9314890
(43) Date de publication de la demande: 14.06.1995
(73) Titulaire: HEMODIA Société Anonyme, F-31450 Fourquevaux (FR)
(72) Inventeur: Montoriol, Pierre, F-31290 Mauremont (FR); Lafaille, Jean Pierre, F-31650 Saint Orens (FR); Aimar, Pierre, F-31400 Toulouse (FR); Montiel, Fabienne, F-31170 Tournefeuille (FR)
(74) Mandataire: Morelle, Guy Georges Alain

(56) Documents cités:
- EP-A- 0 111 696
- EP-A- 0 120 445
- EP-A- 0 246 451
- EP-A- 0 315 252
- EP-A- 0 549 341
- WO-A-91/08782
- US-A- 4 980 056

## Description

La présente invention se rapporte aux domaines concernant le contrôle et la mesure des substances entrant dans la composition des milieux liquides complexes, plus particulièrement aux contrôles et mesures effectués dans le cadre médical, notamment le contrôle et la mesure de la concentration de substances dans le sang lors d'un traitement comme l'hémodialyse.

Il est en effet avantageux de connaître le taux d'une substance déterminée présente par exemple dans le sang, notamment lors d'une séance d'hémodialyse afin de pouvoir ajuster la composition du bain de dialyse en fonction des besoins d'un patient de façon concomitante à la séance de dialyse elle-même. La surveillance et l'ajustement du taux de sodium par exemple sont très important lors de telle séance.

On connaît par la demande de Brevet Européen publiée sous le No. 0 549 394 un procédé pour mesurer la concentration d'une substance dans un milieu complexe liquide circulant dans un corps humain, consistant à mettre en présence, dans un circuit d'écoulement extra-corporel, une fraction du milieu complexe liquide avec un fluide de référence par l'intermédiaire d'une membrane semi-perméable, de manière à obtenir un transfert par diffusion d'une quantité de substance vers le fluide de référence, et à analyser ce dernier pour déterminer la concentration de ladite substance dans le fluide de référence.

Ce procédé présente notamment certains inconvénients. En effet, la présence d'un fluide de référence et le choix du principe de diffusion entraînent notamment l'utilisation de moyens d'agitation du fluide de référence, comme par exemple une pompe de recirculation ou encore des moyens d'agitation mécanique ou ultrasonique. Ces moyens d'agitation et de fluide de référence induisent un coût plus élevé du dispositif permettant de mettre en oeuvre le procédé décrit ci-dessus. D'autre part, malgré l'usage de moyens d'agitation, le principe de diffusion demande un temps de réponse relativement long avant d'obtenir un équilibre osmotique des fluides de part et d'autre de la membrane.

Le document EP-A-0 549 341 qui a pour objet un procédé et un dispositif pour échantillonner un fluide, notamment le sang, lors d'opérations de dialyse en particulier, pallie certains inconvénients de la demande de Brevet Européen citée ci-dessus car l'utilisation d'un fluide de référence est supprimée. L'échantillonneur comprend une enveloppe renfermant un faisceau de fibres creuses formées dans un matériau utilisé pour l'ultrafiltration ou la microfiltration, l'enveloppe entourant le faisceau de fibres. Le fluide dont un échantillon doit être prélevé traverse l'échantillonneur à l'intérieur des fibres, et l'échantillon est prélevé dans l'espace compris entre l'enveloppe et l'extérieur des fibres.

On connaît en outre le document EP-A-0 120 445 qui décrit un procédé et un dispositif de thérapie permettant d'éliminer les composés pathologiques du plasma d'un patient sur un circuit extra-corporel par cryogénie ou chauffage préalable pour obtenir un précipité, puis par filtration et retour au patient du plasma filtré après traitement additionnel éventuellement de ce dernier. La filtration est assuré par gravité au moyen d'une cellule comprenant une enveloppe cylindrique stérile dans laquelle circule le plasma, et une bougie filtrante cylindrique placée dans l'axe de l'enveloppe cylindrique. Le plasma filtré est prélevé à l'intérieur de la bougie tandis que le précipité est retenu à l'extérieur de cette dernière. Cette bougie présente une surface filtrante comprise entre 0,2 et 2 m², sa fonction est la filtration directe du plasma, et son application est thérapeutique directe.

On connaît enfin le document EP-A-0 315 252 qui décrit un dispositif pour séparer le plasma du sang, qui comprend une enveloppe tubulaire dans laquelle est placée un tube contenant une fibre creuse fermée à une de ses extrémités ; une première extrémité de l'enveloppe et du tube est obturée par un bouchon qui permet à une aiguille que l'on y introduit de pénétrer dans l'espace délimité entre l'extérieur de la fibre creuse et les parois intérieures du tube ; la deuxième extrémité de l'enveloppe et du tube est également obturée et porte un bouchon délimitant un espace intérieur en communication avec l'autre extrémité de la fibre creuse de manière à recueillir le plasma. La séparation et le transfert du plasma à l'intérieur de la fibre creuse sont assurés par l'application d'une surpression, lors d'une injection de sang dans le volume compris entre l'extérieur de la fibre creuse et les parois intérieures du tube, supérieure à la pression régnant dans l'espace délimité par l'intérieur de la fibre creuse et le bouchon. La surpression est due au fait que la fibre creuse étant très proche de la paroi intérieure du tube, le sang s'écoule avec un fort taux de cisaillement entraînant une élévation de la pression dans son espace d'écoulement et donc le transfert du plasma à l'intérieur de la fibre creuse.

La présente invention permet en particulier d'éviter ces inconvénients et d'apporter d'autres avantages. Plus précisément, elle consiste en un procédé pour mesurer la concentration d'au moins une substance contenue dans un milieu complexe se déplaçant dans un circuit intérieur à un corps, à partir d'un circuit extérieur au dit corps dérivé dudit circuit intérieur, caractérisé en ce qu'il comprend au moins les étapes suivantes :
* munir préalablement ledit circuit extérieur d'au moins une première enceinte dont les parois intérieures sont étanches au dit milieu complexe, ladite première enceinte étant destinée à être traversée par ledit milieu complexe et comportant des moyens de filtration tangentielle constitués d'au moins une fibre creuse, qui définissent l'enveloppe d'une deuxième enceinte séparée de ladite première enceinte et contenue dans ladite première enceinte,
* établir une pression dudit milieu complexe dans ladite première enceinte supérieure à la pression de gravité et à la pression régnant dans ladite deuxième enceinte pour assurer un transfert par convection au moins d'une fraction de solutés dudit milieu complexe, de ladite première enceinte vers ladite deuxième enceinte,
* analyser au moins ladite fraction de solutés recueillie dans ladite deuxième enceinte et/ou mesurer la concentration d'au moins une substance constituante.

Le procédé selon l'invention permet notamment de supprimer l'utilisation d'un fluide de référence, et de ce fait supprimer tous les inconvénients pré-cités et liés à cette utilisation. En effet, le transfert d'une fraction de solutés dudit milieu complexe de la première enceinte vers la deuxième enceinte s'effectue par convection à travers un moyen de filtration tangentielle, de l'extérieur vers l'intérieur d'au moins une fibre creuse, par l'intermédiaire d'une différence de pression établie entre les première et deuxième enceintes.

La première enceinte peut avantageusement être montée en série sur le circuit extérieur dérivé, et la deuxième enceinte ne comporte avantageusement aucun liquide avant le transfert. La forme de la première enceinte, le choix des moyens de filtration tangentielle qui seront décrits en détail plus loin, et la valeur de la différence de pression permettent notamment de déterminer la rapidité de transfert des solutés. Dès qu'une quantité de solutés minimale pour l'analyse et/ou la mesure selon les besoins a été recueillie dans la deuxième enceinte, des moyens d'analyse et/ou de mesure, qui seront décrits en détail plus loin, peuvent entrer en action. Les moyens de filtration tangentielle peuvent être des moyens de microfiltration ou d'ultrafiltration tangentielle, constitués avantageusement d'au moins une membrane semi-perméable selon le type de solutés et de substances qui doivent être analysés.

Selon une autre caractéristique, le procédé selon l'invention comprend au moins les étapes suivantes :
* munir préalablement ledit circuit extérieur d'au moins une première enceinte dont les parois intérieures sont étanches au dit milieu complexe, ladite première enceinte étant destinée à être traversée par ledit milieu complexe et comportant des moyens de filtration tangentielle constitués d'au moins une fibre creuse, qui définissent l'enveloppe d'une deuxième enceinte séparée de ladite première enceinte et contenue dans ladite première enceinte,
* établir une pression dans ladite deuxième enceinte inférieure à la pression dudit milieu complexe circulant dans ladite première enceinte, par aspiration, pour assurer un transfert par convection au moins d'une fraction de solutés dudit milieu complexe, de ladite première enceinte vers ladite deuxième enceinte,
* analyser au moins ladite fraction de solutés recueillie dans ladite deuxième enceinte et/ou mesurer la concentration d'au moins une substance constituante.

Cette caractéristique permet d'assurer notamment un transfert par convection des solutés, par aspiration dans la deuxième enceinte, ce qui apporte l'avantage de pouvoir monter la première enceinte en un point du circuit extérieur pour lequel la pression du milieu complexe ne serait pas suffisante pour assurer le transfert dans des conditions satisfaisantes.

Selon une autre caractéristique avantageuse, le procédé selon l'invention est constitué d'une combinaison des deux procédés suivant l'invention ci-dessus décrit.

Cette caractéristique permet d'augmenter le différentiel de pression entre les première et deuxième enceintes, et d'optimiser ainsi la vitesse de transfert des solutés et donc le délai nécessaire avant l'analyse de ces derniers.

L'invention a aussi pour objet un dispositif pour mesurer la concentration d'au moins une substance contenue dans un milieu complexe permettant de mettre en oeuvre un procédé selon l'invention, ainsi qu'un module de filtration entrant dans la constitution du dispositif selon l'invention comme défini dans la revendication 15.

D'autres caractéristiques et avantages apparaîtront à la lecture de la description qui suit des exemples de mode de réalisation d'un dispositif selon l'invention, accompagnés des dessins annexés, ces exemples étant donnés à titre d'illustration et sans qu'aucune interprétation restrictive de la présente invention ne puisse en être tirée.
La Figure 1 représente schématiquement un premier exemple de mode de réalisation d'un dispositif selon l'invention monté sur un circuit pour hémodialyse.
La Figure 2 représente schématiquement un deuxième exemple de mode de réalisation d'un dispositif selon l'invention monté sur un circuit pour hémodialyse.
La Figure 3 est une vue détaillée d'un exemple de mode de réalisation d'un module de filtration selon l'invention.

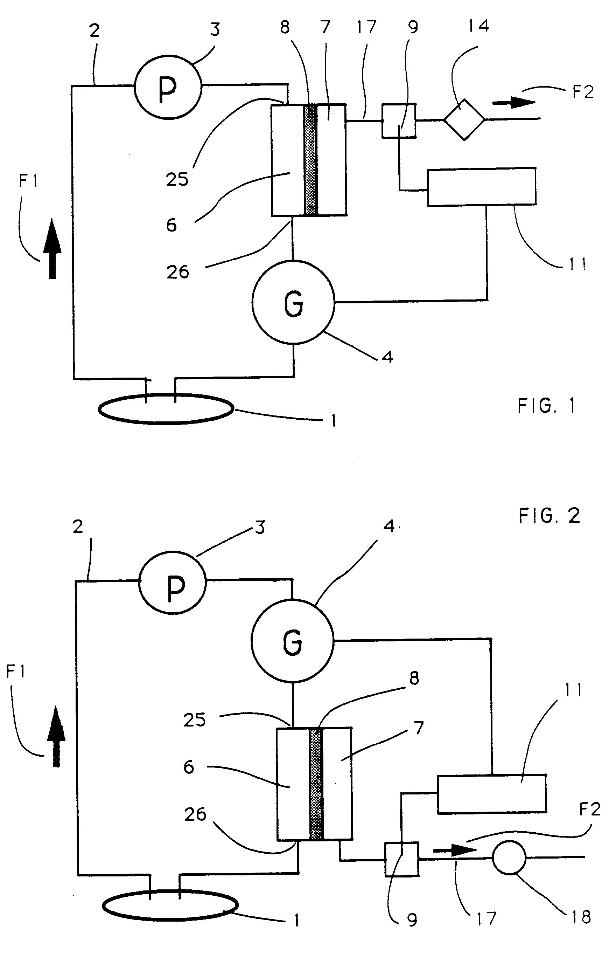

Le circuit extérieur 2 représenté sur la Figure 1 est un circuit schématique pour hémodialyse dérivé à partir d'un circuit intérieur au corps humain 1, comprenant un milieu complexe, en l'occurence le sang, s'écoulant dans le sens représenté par la flèche F1. Ce circuit extérieur comprend notamment de toute manière connue une pompe de circulation 3 du sang, en particulier une pompe péristaltique, et un générateur de dialyse 4 permettant de contrôler les paramètres d'une séance d'hémodialyse. La technique d'hémodialyse étant connue par ailleurs, le générateur de dialyse 4 n'a pas été représenté et ne sera pas décrit plus en détail. Lors d'une séance d'hémodialyse, il est important de connaître très rapidement et de façon continue la concentration de certaines substances, comme par exemple le sodium, dans le sang du patient, afin d'ajuster cette concentration selon les besoins par l'intermédiaire du générateur de dialyse et du liquide de dialyse de toute façon connue.

Le dispositif selon l'invention comprend une première enceinte 6, une deuxième enceinte 7, des moyens de filtration tangentielle formés d'au moins une fibre creuse, avantageusement constituée d'une membrane semi-perméable 8 séparant la première enceinte de la deuxième enceinte, et des moyens d'analyse 9,11 qui seront décrits plus loin. La première enceinte 6 est montée avantageusement en série sur le circuit pour hémodialyse 2 entre la pompe péristaltique 3 et le générateur de dialyse 4, et comprend une entrée 25 et une sortie 26 pour la circulation du sang. La membrane semi-perméable 8 est par exemple réalisée en polysulfone ou acétate de cellulose, ou tout autre matériau permettant d'assurer une microfiltration ou ultrafiltration tangentielle du sang, selon les besoins, de manière à recueillir un perméat dans la deuxième enceinte 7, par l'intermédiaire d'un transfert par convection.

Suivant la Figure 1, la deuxième enceinte 7 est reliée à la pression atmosphérique, avantageusement par l'intermédiaire d'une canalisation 17 et d'un filtre hydrophile 14 en série sur cette dernière, de façon au moins, d'une part, à maintenir une pression sensiblement égale à la pression atmosphérique dans la deuxième enceinte 7 pendant le recueil du perméat et, d'autre part, à éviter une éventuelle contamination, par l'air ambiant, du perméat.

Le dispositif selon l'invention représenté sur la Figure 1 utilise avantageusement la pompe péristaltique 3 pour établir une pression du sang dans la première enceinte 6 supérieure à la pression régnant dans la deuxième enceinte 7, de façon à assurer un transfert par convection au moins d'une fraction de solutés contenus dans le sang, de la première enceinte 6 vers la deuxième enceinte 7.

Le perméat recueilli dans la deuxième enceinte 7, s'écoule par gravité dans une canalisation 17 dans la direction F2 sur la Figure 1, et peut avantageusement être directement analysé sans autre préparation supplémentaire, par l'intermédiaire de moyens d'analyse connus, comprenant au moins un capteur 9 de mesure, par exemple un système d'électrodes de mesures, et une unité 11 de mesure et de traitement informatique, tout moyen d'analyse et/ou de mesure connus pouvant être utilisés en fonction de leurs sensibilité et performances à l'égard des substances pour lesquelles on désire connaître la concentration . Le capteur 9 est notamment placé dans la deuxième enceinte 7, ou comme le montre la Figure 1 le plus proche possible de cette dernière, et l'unité 11 peut avantageusement être connectée au générateur de dialyse 4, comme le représente la Figure 1, afin d'ajuster la concentration de substances dans le liquide de dialyse en fonction des résultats de mesure donnés par l'unité 11.

Des essais ont été effectués, montrant que le transfert par convection essentiel au dispositif selon l'invention permet d'obtenir très rapidement notamment une fraction de solutés du milieu complexe, dans des temps de l'ordre de quelques minutes et moins selon notamment la surface et le coefficient de perméabilité de la membrane semi-perméable utilisée. D'autre part, on notera que l'analyse se fait directement à partir d'une fraction du milieu complexe et non pas à partir d'un fluide de référence dans lequel les substances à analyser se diffusent, comme c'est le cas notamment avec le dispositif décrit dans la demande de Brevet No. 0 549 394 déjà citée. De ce fait, les risques éventuels de contamination du sang par le fluide de référence sont écartés, le coût de revient du dispositif selon l'invention est amélioré ainsi que sa sécurité d'emploi, et les résultats d'analyse et/ou de mesure sont plus simples à obtenir.

On notera que dans le cas ou le circuit extérieur 2 ne dispose pas d'une pression suffisante pour établir un transfert par convection à travers la membrane 8, le dispositif selon l'invention peut éventuellement comprendre tout moyen de pompe connu et approprié selon les besoins palliant cet inconvénient.

Sur la Figure 2, on a représenté schématiquement un circuit extérieur pour hémodialyse 2 similaire à celui de la Figure 1, dans lequel le dispositif selon l'invention y est avantageusement monté de façon analogue, mais est maintenant positionné en aval du générateur de dialyse 4 dans le sens d'écoulement du sang représenté par la flèche F1. Dans cette partie du circuit 2, en aval du générateur de dialyse 4, en pratique il peut se faire que la pression du milieu complexe ne soit pas suffisante pour assurer un transfert par convection d'un perméat à travers la membrane semi-perméable 8. De ce fait, le dispositif selon l'invention comprend avantageusement des moyens d'aspiration 18 additionnels pour générer ledit transfert.

Les moyens d'aspiration comprennent avantageusement une pompe 18, par exemple une pompe péristaltique, reliée par une canalisation 17 à la deuxième enceinte 7 afin d'établir une pression dans celle-ci inférieure à la pression du sang circulant dans la première enceinte 6, par aspiration, pour assurer un transfert par convection au moins d'une fraction de solutés contenus dans le sang, de la première enceinte 6 vers la deuxième enceinte 7.

Le capteur 9 pour une analyse du perméat peut avantageusement être placé dans la deuxième enceinte 7 ou comme représenté sur la Figure 2 dans la canalisation 17, et le plus proche possible de la deuxième enceinte 7 afin de minimiser le trajet du perméat pour arriver sur le capteur 9, et donc le temps de réponse du dispositif selon l'invention.

Le capteur 9 est relié à une unité de mesure et de traitement informatique 11, elle-même reliée avantageusement au générateur de dialyse 4 pour les raisons déjà citées.

Il sera évident à un homme de l'art d'utiliser tout autre moyen d'aspiration connu afin d'établir un transfert de perméat à travers la membrane 8 notamment lorsque la pression dans première enceinte 6 est voisine de celle régnant dans la deuxième enceinte 7.

Il est à noter que la canalisation 17 peut comprendre un moyen de filtre (non représenté), par exemple un filtre hydrophile, en particulier afin d'éviter toute éventuelle contamination du perméat par l'air ambiant extérieur.

On relèvera que le dispositif selon l'invention peut comprendre une combinaison des moyens décrits sur les Figures 1 et 2. C'est à dire que l'on peut, selon notamment la vitesse de transfert désirée, par exemple munir le dispositif représenté sur la Figure 1 de moyens d'aspiration dans la deuxième enceinte 7, en particulier ceux montrés sur la Figure 2, de façon à augmenter le différentiel de pression entre les première et deuxième enceintes.

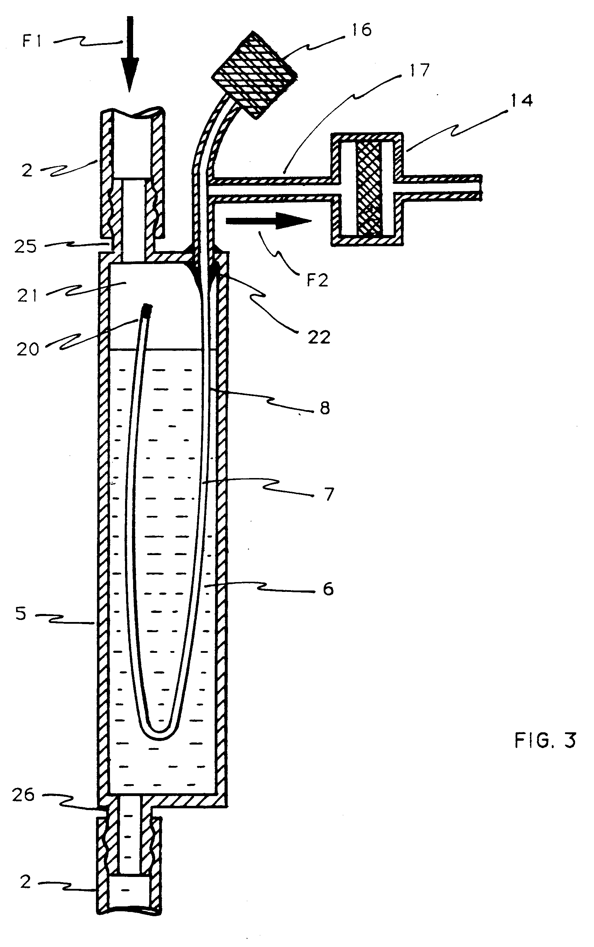

La Figure 3 représente un exemple détaillé de mode de réalisation d'un module de filtration selon l'invention entrant dans la constitution d'un dispositif selon l'invention qui peut être monté sur un circuit extérieur d'hémodialyse conventionnel. Le dispositif comprend, une enveloppe 5, avantageusement de forme tubulaire, dont l'intérieur définit une première enceinte 6, et possédant une entrée 25 et une sortie 26 pour la circulation du sang dans le sens de la flèche F1, au moins une fibre creuse 8 comme membrane semi-perméable, placée dans l'enveloppe 5, et dont l'intérieur définit une deuxième enceinte 7 séparée de la première enceinte par la paroi de la fibre creuse avantageusement comme suit: une première extrémité 20 de la fibre creuse est obturée et libre dans l'enveloppe 5, et une deuxième extrémité 22 de la fibre creuse est reliée de toute façon connue à l'enveloppe 5 de façon que l'intérieur de la fibre creuse soit en communication avec une canalisation extérieure 17, pour l'écoulement du perméat, la canalisation 17 étant avantageusement comprise dans le dispositif selon l'invention. Le dispositif comprend notamment un filtre hydrophile 14 en série sur la canalisation 17, et un site de prélèvement 16 monté de façon à permettre un accés, par exemple au moyen d'une aiguille de seringue, dans la canalisation 17. Tous moyens de capteur de mesure (non représentés) peuvent être notamment insérés entre l'extrémité 22 de sortie de fibres creuses et le filtre 14.

La disposition de la ou des fibres creuses 8 dans l'enveloppe 5 sera effectuée de manière telle qu'une surface extérieure maximale de la fibre creuse soit au contact de lignes de courant de l'écoulement du sang dans la première enceinte 6 pour éviter dans le cas contraire une obstruction trop rapide de la ou des fibres creuses par de grosses molécules contenues dans le sang. A cet effet, et comme représenté sur la Figure 3, la ou les fibres creuses peuvent adopter une forme linéaire double avec un coude proche d'une extrémité de l'enveloppe 5, ou toute autre forme, par exemple hélicoïdale ou linéaire simple, de manière à obtenir une filtration tangentielle maximale suivant les lignes de courant.

Une variante (non représentée) du dispositif consiste à relier la première extrémité 20 de la fibre creuse à l'enveloppe 5 pour établir un écoulement du perméat dans la canalisation 17 à la fois par les première et deuxième extrémités de la fibre creuse 8.

Le perméat recueilli à l'intérieur de la ou des fibres creuses par transfert par convection s'achemine dans la direction F2, par gravité ou par aspiration dans la canalisation 17 où il passe sur au moins un capteur de mesure (non représenté), puis est rejeté à l'extérieur.

Comme cela est représenté sur la Figure 3, la première enceinte 6 peut être utilisée comme un piège à bulles nécessaire dans un circuit extérieur pour hémodialyse. En effet, l'enveloppe 5 peut adopter la forme d'un piège à bulles conventionnel de façon à réserver un espace gazeux 21 en partie supérieure de la première enceinte.

Il est à noter que l'insertion avantageuse d'un site de prélèvement 16 donne la possibilité à un opérateur de prélever un échantillon de perméat, par exemple en cours de dialyse, au moyen d'une seringue, pour être analysé avec tout moyen autonome approprié de laboratoire selon les besoins.

On remarquera la simplicité du dispositif selon l'invention, sa facilité de mise en oeuvre notamment avec des matériaux et techniques compatibles à un contact sanguin le cas échéant, matériaux et techniques largement répandus dans l'industrie des instruments, outils, et accessoires médicaux. On notera en conséquence la possibilité d'une fabrication en grande série à un coût relativement peu élevé, et d'une destination d'usage unique du dispositif selon l'invention, dans la plus grande sécurité.

## Revendications

1. Procédé pour mesurer la concentration d'au moins une substance contenue dans un milieu complexe se déplaçant dans un circuit intérieur à un corps (1), à partir d'un circuit extérieur (2) au dit corps dérivé dudit circuit intérieur,
***caractérisé en ce qu***'il comprend au moins les étapes suivantes :
* munir préalablement ledit circuit extérieur d'au moins une première enceinte (6) dont les parois intérieures sont étanches au dit milieu complexe, ladite première enceinte étant destinée à être traversée par ledit milieu complexe et comportant des moyens de filtration tangentielle constitués d'au moins une fibre creuse (8), qui définissent l'enveloppe d'une deuxième enceinte (7) séparée de ladite première enceinte et contenue dans ladite première enceinte,
* établir une pression dudit milieu complexe dans ladite première enceinte supérieure à la pression de gravité et à la pression régnant dans ladite deuxième enceinte pour assurer un transfert par convection au moins d'une fraction de solutés dudit milieu complexe, de ladite première enceinte vers ladite deuxième enceinte,
* analyser au moins ladite fraction de solutés recueillie dans ladite deuxième enceinte et/ou mesurer la concentration d'au moins une substance constituante.

2. Procédé pour mesurer la concentration d'au moins une substance contenue dans un milieu complexe se déplaçant dans un circuit intérieur à un corps, à partir d'un circuit extérieur (2) au dit corps dérivé dudit circuit intérieur, ***caractérisé en ce qu***'il comprend au moins les étapes suivantes :
* munir préalablement ledit circuit extérieur d'au moins une première enceinte (6) dont les parois intérieures sont étanches au dit milieu complexe, ladite première enceinte étant destinée à être traversée par ledit milieu complexe et comportant des moyens de filtration tangentielle constitués d'au moins une fibre creuse (8), qui définissent l'enveloppe d'une deuxième enceinte (7) séparée de ladite première enceinte et contenue dans ladite première enceinte,
* établir une pression dans ladite deuxième enceinte inférieure à la pression dudit milieu complexe circulant dans ladite première enceinte, par aspiration, pour assurer un transfert par convection au moins d'une fraction de solutés dudit milieu complexe, de ladite première enceinte vers ladite deuxième enceinte,
* analyser au moins ladite fraction de solutés recueillie dans ladite deuxième enceinte et/ou mesurer la concentration d'au moins une substance constituante.

3. Procédé ***caractérisé en ce qu***'il est constitué d'une combinaison des procédés suivant les revendications 1 et 2.

4. Procédé suivant la revendication 1, 2, ou 3, ***caractérisé en ce que*** ledit corps est constitué par un corps humain (1), ledit milieu complexe comprenant du sang.

5. Procédé suivant la revendication 4, ***caractérisé en ce que*** ledit circuit extérieur dérivé comprend un circuit sanguin (2) extra-corporel, par exemple pour hémodialyse.

6. Procédé suivant la revendication 4 ou 5, ***caractérisé en ce qu***'il consiste à utiliser ladite première enceinte (6) comme un piège à bulles.

7. Procédé suivant les revendications 1 et 5 ou les revendications 1 et 6, ***caractérisé en ce que*** ladite pression supérieure dans ladite première enceinte (6) par rapport à celle régnant dans ladite deuxième enceinte (7), est assurée par une pompe (3) de circulation du sang dudit circuit pour hémodialyse (2).

8. Dispositif pour mesurer la concentration d'au moins une substance contenue dans un milieu complexe permettant de mettre en oeuvre un procédé suivant l'une des revendications 1 à 7, ***caractérisé en ce qu***'il comprend, une première enceinte (6) dont les parois intérieures sont étanches au dit milieu complexe et comportant une entrée (25) et une sortie (26) pour un écoulement continu dudit milieu complexe, une deuxième enceinte (7) séparée de ladite première enceinte et contenue dans ladite première enceinte, au moins une fibre creuse (8), pour une séparation desdites première et deuxième enceintes, définissant l'enveloppe de ladite deuxième enceinte (7), des moyens pour établir une différence de pression entre lesdites première et deuxième enceintes pour permettre un transfert par convection au moins d'une fraction de solutés contenus dans ledit milieu complexe, de ladite première enceinte vers ladite deuxième enceinte, des moyens (9, 11) d'analyse et/ou de mesure au moins de ladite fraction de solutés recueillie dans ladite deuxième enceinte.

9. Dispositif suivant la revendication 8*, **caractérisé en ce qu***'une première des extrémités (20) de ladite fibre creuse (8) est reliée à une enveloppe (5) dont l'intérieur définit ladite première enceinte (6), la deuxième extrémité (22) de ladite fibre creuse (8) étant en outre reliée à ladite enveloppe (5), pour établir un écoulement de ladite fraction de solutés dans une canalisation (17) à la fois par lesdites première et deuxième extrémités de la fibre creuse (8).

10. Dispositif suivant la revendication 8, ***caractérisé en ce que*** ladite fibre creuse (8) est obstruée à une première de ses extrémités (20).

11. Dispositif suivant l'une quelconque des revendications 8 à 10, ***caractérisé en ce que*** ladite première enceinte adopte une forme de piège à bulles.

12. Dispositif suivant l'une quelconque des revendications 8 à 11, ***caractérisé en ce que*** ladite deuxième enceinte (7) est en communication avec des moyens de site de prélèvement (16).

13. Dispositif suivant l'une quelconque des revendications 8 à 12, ***caractérisé en ce que*** lesdits moyens pour établir une différence de pression entre lesdites première et deuxième enceintes comprennent des moyens de pompe (3) pour établir une pression dudit milieu complexe dans ladite première enceinte (6) supérieure à celle régnant dans ladite deuxième enceinte (7).

14. Dispositif suivant l'une quelconque des revendications 8 à 13, ***caractérisé en ce que*** lesdits moyens pour établir une différence de pression entre lesdites première et deuxième enceintes comprennent des moyens d'aspiration (18) pour établir une pression dans ladite deuxième enceinte (7) inférieure à la pression dudit milieu complexe dans ladite première enceinte (6).

15. Module de filtration entrant dans la constitution d'un dispositif suivant l'une des revendications 8 à 14, ***caractérisé en ce qu***'il comprend, une première enceinte (6) dont les parois intérieures sont étanches au dit milieu complexe et comportant une entrée (25) et une sortie (26) pour un écoulement continu dudit milieu complexe, une deuxième enceinte (7) séparée de ladite première enceinte et contenue dans ladite première enceinte, au moins une fibre creuse (8), pour une séparation desdites première et deuxième enceintes, définissant l'enveloppe de ladite deuxième enceinte (7), pour permettre un transfert par convection au moins d'une fraction de solutés contenus dans ledit milieu complexe, de ladite première enceinte vers ladite deuxième enceinte.

## Claims

1. Method of measuring the concentration of at least one substance contained in a complex medium moving into an internal circuit inside a body (1) from an external circuit (2) outside said body derived from said internal circuit, characterised in that it comprises at least the following steps:
* providing said external circuit in advance with at least a first chamber (6), the interior walls of which are impervious to said complex medium, said first chamber being intended to be traversed by said complex medium and comprising tangential filtration means, which are formed from at least one hollow fibre (8), and which define the casing of a second chamber (7) separated from said first chamber and contained in said first chamber,
* establishing a pressure of said complex medium in said first chamber higher than the pressure of gravity and the pressure prevailing in said second chamber to ensure a transfer by convection of at least a fraction of solutes of said complex medium from said first chamber to said second chamber,
* analysing at least said fraction of solutes collected in said second chamber and/or measuring the concentration of at least one constituent substance.

2. Method of measuring the concentration of at least one substance contained in a complex medium moving into an internal circuit inside a body from an external circuit (2) outside said body derived from said internal circuit, characterised in that it comprises at least the following steps:
* providing said external circuit in advance with at least a first chamber (6), the interior walls of which are impervious to said complex medium, said first chamber being intended to be traversed by said complex medium and comprising tangential filtration means, which are formed from at least one hollow fibre (8) and which define the casing of a second chamber (7) separated from said first chamber and contained in said first chamber,
* establishing a pressure in said second chamber lower than the pressure of said complex medium circulating in said first chamber, by suction, to ensure a transfer by convection of at least a fraction of solutes of said complex medium from said first chamber to said second chamber,
* analysing at least said fraction of solutes collected in said second chamber and/or measuring the concentration of at least one constituent substance.

3. Method characterised in that it comprises a combination of the methods according to claims 1 and 2.

4. Method according to claim 1, 2 or 3, characterised in that said body consists of a human body (1), said complex medium comprising blood.

5. Method according to claim 4, characterised in that said derived external circuit comprises an extra-corporal blood circuit (2), for example for haemodialysis.

6. Method according to claim 4 or 5, characterised in that it comprises using said first chamber (6) as a bubble trap.

7. Method according to claims 1 and 5 or claims 1 and 6, characterised in that said higher pressure in said first chamber (6), compared with that prevailing in said second chamber (7), is ensured by a circulation pump (3) circulating the blood of said circuit for haemodialysis (2).

8. Apparatus for measuring the concentration of at least one substance contained in a complex medium, which apparatus permits a method according to one of claims 1 to 7 to be accomplished, characterised in that it comprises a first chamber (6), the interior walls of which are impervious to said complex medium, and which chamber comprises an inlet (25) and an outlet (26) for a continuous flow of said complex medium, a second chamber (7) separated from said first chamber and contained in said first chamber, at least one hollow fibre (8) for separating said first and second chambers and defining the casing of said second chamber (7), means for establishing a pressure difference between said first and second chambers to permit a transfer by convection of at least a fraction of solutes contained in said complex medium from said first chamber to said second chamber, and means (9, 11) for analysing and/or for measuring at least said fraction of solutes collected in said second chamber.

9. Apparatus according to claim 8, characterised in that a first end (20) of said hollow fibre (8) is connected to a casing (5), the interior of which defines said first chamber (6), the second end (22) of said hollow fibre (8) also being connected to said casing (5), to establish a flow of said fraction of solutes into a line (17) through said first and second ends of the hollow fibre (8) at the same time.

10. Apparatus according to claim 8, characterised in that said hollow fibre (8) is blocked at a first of its ends (20).

11. Apparatus according to any of claims 8 to 10, characterised in that said first chamber is configured as a bubble trap.

12. Apparatus according to any of claims 8 to 11, characterised in that said second chamber (7) communicates with sample removing means (16).

13. Apparatus according to any of claims 8 to 12, characterised in that said means for establishing a pressure difference between said first and second chambers comprise pumping means (3) for establishing a pressure of said complex medium in said first chamber (6) higher than the pressure prevailing in said second chamber (7).

14. Apparatus according to any of claims 8 to 13, characterised in that said means for establishing a pressure difference between said first and second chambers comprise suction means (18) for establishing a pressure in said second chamber (7) lower than the pressure of said complex medium in said first chamber (6).

15. Filtration module incorporated in the composition of an apparatus according to one of claims 8 to 14, characterised in that it comprises a first chamber (6), the interior walls of which are impervious to said outlet (26) for a continuous flow of said complex medium, a second chamber (7) separated from said first chamber and contained in said first chamber, at least one hollow fibre (8) for separating said first and second chambers and for defining the casing of said second chamber (7), to permit a transfer by convection of at least a fraction of solutes contained in said complex medium from said first chamber to said second chamber.

## Patentansprüche

1. Verfahren zur Messung der Konzentration von mindestens einer Substanz, die in einem komplexen Medium enthalten ist, welches sich in einem inneren Kreislauf eines Körpers (1), ausgehend von einem außerhalb des Körpers befindlichen Kreislauf (2) verlagert, der von dem inneren Kreislauf abgezweigt ist,
**dadurch gekennzeichnet, daß** es mindestens folgende Schritte umfaßt:
* zunächst Versehen des äußeren Kreislaufs mit mindestens einer ersten Hülle (6), deren innere Wände gegenüber dem komplexen Medium dicht sind, wobei die erste Hülle dazu bestimmt ist, von dem komplexen Medium durchdrungen zu werden, und Mittel zum tangentialen Filtern aufweist, die aus mindestens einer hohlen Faser (8) gebildet sind, die den Mantel einer zweiten Hülle (7) festlegen, die von der ersten Hülle getrennt und in der ersten Hülle enthalten ist,
* Erzeugen eines Drucks des komplexen Mediums in der ersten Hülle, der größer als der Schwerkraftdruck und als der Druck ist, der in der zweiten Hülle herrscht, um durch Konvektion einen Transfer von mindestens einer Fraktion gelöster Stoffe des komplexen Mediums, von der ersten Hülle in Richtung auf die zweite Hülle zu gewährleisten,
* Analysieren mindestens der Fraktion gelöster Stoffe, die in der zweiten Hülle gesammelt ist, und/oder Messen der Konzentration von mindestens einer eine Komponente bildenden Substanz.

2. Verfahren zur Messung der Konzentration von mindestens einer Substanz, die in einem komplexen Medium enthalten ist, welches sich in einem inneren Kreislauf eines Körpers, ausgehend von einem außerhalb des Körpers befindlichen Kreislauf (2) verlagert, der von dem inneren Kreislauf abgezweigt ist,
**dadurch gekennzeichnet, daß** es mindestens folgende Schritte umfaßt;
* zunächst Versehen des äußeren Kreislaufs mit mindestens einer ersten Hülle (6), deren innere Wände gegenüber dem komplexen Medium dicht sind, wobei die erste Hülle dazu bestimmt ist, von dem komplexen Medium durchdrungen zu werden und Mittel zum tangentialen Filtern aufweist, die aus mindestens einer hohlen Faser (8) gebildet sind, die den Mantel einer zweiten Hülle (7) festlegen, die von der ersten Hülle getrennt und in der ersten Hülle enthalten ist,
* Erzeugen eines Drucks durch Ansaugen in der zweiten Hülle, der niedriger als der Druck des komplexen Mediums ist, welches in der ersten Hülle zirkuliert, um durch Konvektion einen Transfer von mindestens einer Fraktion gelöster Stoffe des komplexen Mediums, von der ersten Hülle in Richtung auf die zweite Hülle zu gewährleisten,
* Analysieren mindestens der Fraktion gelöster Stoffe, die in der zweiten Hülle gesammelt ist, und/oder Messen der Konzentration von mindestens einer eine Komponente bildenden Substanz.

3. Verfahren, **dadurch gekennzeichnet, daß** es aus einer Kombination der Verfahren nach Anspruch 1 und 2 besteht.

4. Verfahren nach Anspruch 1, 2 oder 3,
**dadurch gekennzeichnet, daß** der Körper aus einem menschlichen Körper (1) gebildet ist, wobei das komplexe Medium Blut enthält.

5. Verfahren nach Anspruch 4,
**dadurch gekennzeichnet, daß** der abgezweigte äußere Kreislauf einen außerhalb des menschlichen Körpers bestehenden Blutkreislauf (2), beispielsweise zur Hämodialyse, umfaßt.

6. Verfahren nach Anspruch 4 oder 5,
**dadurch gekennzeichnet, daß** es darin besteht, die erste Hülle (6) als eine Blasenfalle zu verwenden.

7. Verfahren nach Anspruch 1 und 5 oder Anspruch 1 und 6, **dadurch gekennzeichnet, daß** der höhere Druck in der ersten Hülle (6) bezogen auf denjenigen, welcher in der zweiten Hülle (7) herrscht, durch eine Pumpe (3) zum Umlaufen (3) des Bluts des Hämodialysekreislaufs (2) gewährleistet wird.

8. Vorrichtung zur Messung der Konzentration von mindestens einer Substanz, die in einem komplexen Medium enthalten ist, welche Vorrichtung gestattet, ein Verfahren nach einem der Ansprüche 1 bis 7 auszuführen,
**dadurch gekennzeichnet, daß** sie eine erste Hülle (6), deren innere Wände gegenüber dem komplexen Medium dicht sind und die einen Einlaß (25) und einen Auslaß (26) für einen kontinuierlichen Abfluß des komplexen Mediums aufweist, eine zweite Hülle (7), die von der ersten Hülle getrennt und in der ersten Hülle enthalten ist, mindestens eine hohle Faser (8) zum Trennen der ersten und zweiten Hülle, die den Mantel der zweiten Hülle (7) festlegt, Mittel zum Erzeugen eines Druckunterschieds zwischen der ersten und der zweiten Hülle, um durch Konvektion einen Transfer von mindestens einer Fraktion gelöster Stoffe, die in dem komplexen Medium enthalten sind, von der ersten Hülle in Richtung auf die zweite Hülle zu gestatten, Mittel (9, 11) zum Analysieren und/oder Messen von mindestens der Fraktion gelöster Stoffe, die in der zweiten Hülle gesammelt sind, umfaßt.

9. Vorrichtung nach Anspruch 8,
**dadurch gekennzeichnet, daß** ein erstes Ende der Enden (29) der hohle Faser (8) mit einem Mantel (5) verbunden ist, dessen Innenraum die erste Hülle (6) festlegt, wobei das zweite Ende (22) der hohlen Faser (8) außerdem mit dem Mantel (5) verbunden ist, um ein Anfließen der Fraktion gelöster Stoffe in eine Leitung (17) gleichzeitig durch das erste und zweite Ende der hohlen Faser (8) zu erzeugen.

10. Vorrichtung nach Anspruch 8,
**dadurch gekennzeichnet, daß** die hohle Faser (8) an einem ersten Ende seiner Enden (20) verstopft ist.

11. Vorrichtung nach irgendeinem der Ansprüche 8 bis 10, **dadurch gekennzeichnet, daß** die erste Hülle eine Form einer Blasenfalle annimmt.

12. Vorrichtung nach irgendeinem der Ansprüche 8 bis 11, **dadurch gekennzeichnet, daß** die zweite Hülle (7) mit den Mitteln (16) des Entnahmeorts in Verbindung steht.

13. Vorrichtung nach irgendeinem der Ansprüche 8 bis 12, **dadurch gekennzeichnet, daß** die Mittel zur Erzeugung eines Druckunterschieds zwischen der ersten und zweiten Hülle Pumpenmittel (3) umfassen, um einen Druck des komplexen Mediums in der ersten Hülle (6) zu erzeugen, der höher als derjenige ist, welcher in der zweiten Hülle (7) herrscht.

14. Vorrichtung nach irgendeinem der Ansprüche 8 bis 13, **dadurch gekennzeichnet, daß** die Mittel zur Erzeugung eines Druckunterschieds zwischen der ersten und zweiten Hülle Ansaugmittel (18) umfassen, um einen Druck in der zweiten Hülle (7) zu erzeugen, der niedriger als der Druck des komplexen Mediums in der ersten Hülle (6) ist.

15. Filtriermodul, das in den Aufbau einer Vorrichtung nach einem dem Ansprüche 8 bis 14 eingeht,
**dadurch gekennzeichnet, daß** es eine erste Hülle (6), deren innere Wände gegenüber dem komplexen Medium dicht sind und die einen Einlaß (25) und einen Auslaß (26) für einen kontinuierlichen Abfluß des komplexen Mediums aufweist, eine zweite Hülle (7), die von der ersten Hülle getrennt und in der ersten Hülle enthalten ist, mindestens eine hohle Faser (8) für eine Trennung der ersten und zweiten Hülle, welche hohle Faser (8) den Mantel der zweiten Hülle (7) festlegt, um durch Konvektion einen Transfer von mindestens einer Fraktion gelöster Stoffe, die in dem komplexen Medium enthalten sind, von der ersten Hülle in Richtung auf die zweite Hülle zu gestatten, umfaßt.
